# EUROPEAN PATENT SPECIFICATION

(11) **EP 4 285 894 B1**
(45) Date of publication and mention of the grant of the patent: **21.10.2026**
(21) Application number: 23174796.5
(22) Date of filing: 23.05.2023
(51) Int. Cl.: A61K 9/20, A61K 47/12

(54) **A FORMULATION OF EMPAGLIFLOZIN AND METFORMIN HYDROCHLORIDE**
FORMULIERUNG VON EMPAGLIFLOZIN UND METFORMINHYDROCHLORID
FORMULATION D'EMPAGLIFLOZINE ET DE CHLORHYDRATE DE METFORMINE

(30) Priority: 31.05.2022 TR 202208869; 15.06.2022 TR 202209903
(43) Date of publication of application: 06.12.2023
(73) Proprietor: Sanovel Ilac Sanayi ve Ticaret A.S., Sisli - Istanbul (TR)
(72) Inventor: GULER, Tolga, Istanbul (TR); ARMUT, Merve, Istanbul (TR); PEHLIVAN AKALIN, Nur, Istanbul (TR); SUNEL, Fatih, Istanbul (TR)
(74) Representative: Genç Ilhan, Oznur

(56) References cited:
- WO-A1-2020/058095
- WO-A1-2021/123165
- CN-A- 114 404 436

## Description

### Field of the Invention

The present invention relates to a film coated tablet comprising an intragranular composition comprising empagliflozin and metformin hydrochloride and at least one binder, wherein the intragranular composition is obtained by wet-granulation using a solvent comprising alcohol. The process is simple, rapid, cost effective, time-saving and industrially convenient process.

### Background of the Invention

Diabetes mellitus is a group of disorders of carbohydrate metabolism in which the action of insulin is diminished or absent through altered secretion, decreased insulin activity or a combination of both factors. There are two main types of diabetes; Type 1 and Type 2: Type 1 diabetes occurs because the insulin-producing cells of the pancreas (beta cells) are damaged. In Type 1 diabetes, the pancreas makes little or no insulin, so sugar cannot get into the body's cells for use as energy. People with Type 1 diabetes must use insulin injections to control their blood glucose. In Type 2 diabetes, the pancreas makes insulin, but it either doesn't produce enough, or the insulin does not work properly. This diabetes occurs most often in people who are over 40 years old and overweighed. Type 2 diabetes may sometimes be controlled with a combination of diet, weight management, and exercise. However, treatment also may include oral glucose-lowering medications or insulin injections.

Metformin is antidiabetics having an orally-administrated biguanide structure. Metformin hydrochloride is a white to off-white crystalline compound and it is freely soluble in water and practically insoluble in acetone, ether and chloroform. Oral doses of metformin are generally recommended in the range of 500 to 2500 mg a day and a single dose may vary from 500 to 850 mg. It is used singly or in combination with sulfonylureas, alpha-glucosidase inhibitors, or insulin.

The chemical name of metformin hydrochloride is 1,1-dimethylbiguanide hydrochloride, has the following chemical structure of Formula I.

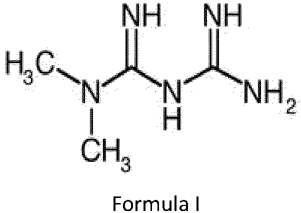

Empagliflozin is a known SGLT2 inhibitor that is described for the treatment or improvement in glycemic control in patients with type 2 diabetes mellitus. The chemical name of empagliflozin is 1 - chloro-4-(3-D-glucopyranos-1 -yl)- 2-[4-((S)-tetrahydrofuran-3-yloxy)-benzyl]-benzene and its chemical structure is shown in the Formula II.

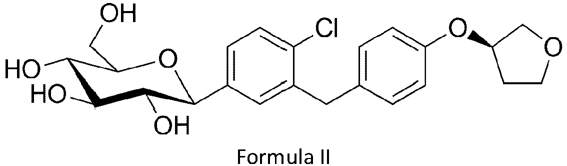

Combination product of empagliflozin and metformin hydrochloride is marketed under the trademark Synjardy^{®}. The combination is to help control blood glucose in people with T2D. Empagliflozin, a sodium glucose co-transporter-2 (SGLT2) inhibitor, removes excess glucose through the urine by blocking glucose re-absorption in the kidney.

Active ingredients have some disadvantages in the formulation and process. The main problem encountered when preparing formulations comprising empagliflozin is low solubility, leading to difficulties with disintegration and dissolution times. Furthermore, metformin is a very poorly compressible active substance and metformin presents in high amounts in a composition. This causes some problems for examples; homogeneity, flowability and dissolution profile.

WO2011039337 (A1) application discloses pharmaceutical compositions comprising fixed dose combinations of a SGLT-2 inhibitor drug and a partner drug, processes for the preparation thereof, and their use to treat certain diseases.

CN104586834 (A) application discloses a pharmaceutical composition of empagliflozin and metformin, a preparation method and application thereof. The composition comprises the following components: i.) empagliflozin; ii.) metformin hydrochloride; and one or more fillers; one or more adhesives; one or more flow aids and one or more lubricants.

In the prior art, there are also several patents which disclose empagliflozin and metformin hydrochloride in oral pharmaceutical dosage forms. However, an effective formulation and method has not been disclosed.

WO 2021/123165 A1 discloses a solid pharmaceutical dosage form comprising an amorphous solid solution of empagliflozin with at least one polymer, and exemplifies a film coated tablet comprising empagliflozin in combination with metformin hydrochloride and magnesium stearate as lubricant. WO 2021/123165 A1 focuses on providing pharmaceutical compositions comprising empagliflozin, which are which are physically and chemically stable, have improved dissolution profile and are prepared by an economical process suitable for use on a commercial scale.

WO 2020/058095 A1 relates to stable pharmaceutical compositions of empagliflozin, in the form of immediate release tablets and to a process for the manufacture of said stable pharmaceutical compositions. The aim of WO 2020/058095 A1 is to provide a pharmaceutical composition with a good dissolution profile that enables a high bioavailability of empagliflozin in a patient, while it avoids or reduces filming and sticking during the manufacturing process of the composition.

CN 114 404 436 A belongs to the field of medicine, in particular to a metformin empagliflozin composition and preparation method thereof.

There still remains a need in the art to provide an improved a film coated tablet formulation comprising empagliflozin and metformin hydrochloride having high solubility, excellent pharmacomechanic properties and accordingly a high bioavailability and a long-term stability which is also obtained by using an effective process.

### Detailed Description of the Invention

The main object of the present invention is to provide a film coated tablet comprising empagliflozin and metformin hydrochloride with excellent physicochemical properties, such as compressibility, flowability, homogeneity, and content uniformity which overcomes the above-described problems in the prior art and have additive advantages over them.

Another object of the present invention is to provide a film coated tablet comprising empagliflozin and metformin hydrochloride with having the desired level of dissolution rate and high stability. Another object of the present invention is to provide a process for preparing a film coated tablet comprising empagliflozin and metformin hydrochloride. The process is a simple, rapid, cost effective, time-saving, and industrially convenient method.

As used herein, the term "intragranular composition" refers to a population of granules that are wetted with solvent and then dried and granulated. Intragranular composition can be called also "granulate" or "granulate component". The "extragranular composition" refers is the part that does not participate in wet granulation.

The main problem encountered when preparing formulations comprising empagliflozin is low solubility, leading to difficulties with disintegration and dissolution times. Furthermore, metformin is a very poorly compressible active substance, poor flowability and metformin presents in high amounts in a composition. This causes some problems for examples; compressibility, homogeneity, flowability and dissolution profile. Therefore, the excipients and process steps used are very important.

Physicochemical properties are important parameters in tablet formulation. It can be a problem especially in formulations containing a high amount of active substance. In a formulation, a problem of physicochemical properties with these negatively affects the dissolution profile and stability. In this formulation, wet granulation is very important since both metformin HCl has a high amount and metformin already has flowability and compressibility problems. We have found in this invention that wet granulation with a solvent comprising alcohol overcomes these problems.

According to an embodiment of the present invention, a film coated tablet comprises an intragranular composition comprising empagliflozin and metformin hydrochloride at least one binder, wherein the intragranular composition is obtained by wet-granulation using a solvent comprising alcohol. We saw that the use of the binder surprisingly achieved content uniformity, the desired flowability and homogeneity of the active agent. Encountered while developing formulations is the compressibility of metformin HCl, which makes the production difficult. The used process provides to overcome this problem.

Suitable binders are selected from the group comprising hydroxypropyl cellulose, hydroxypropyl methylcellulose, pregelatinized starch, polyvinylpyrrolidone, carboxymethylcellulose sodium, cellulose acetate phthalate, chitosan, dextrose, ethylcellulose, glyceryl behenate, hydroxyethyl cellulose, hydroxyethylmethyl cellulose, hydroxypropyl starch, magnesium aluminum silicate, poloxamer, polycarbophil, polydextrose, polyethylene oxide, polymethacrylates, polyoxyethilene-alkyl ethers or mixtures thereof.

According to an embodiment of the present invention, the binder is hydroxypropyl methyl cellulose or hydroxypropyl cellulose. Preferably, the binder is hydroxypropyl cellulose.

According to an embodiment of the present invention, the amount of binders is 2.0% to 15.0% by weight in the total composition. Preferably, the amount of binders is 2.0% to 10.0% by weight in the total composition.

According to an embodiment of the present invention, the amount of metformin hydrochloride is 70.0% to 90.0% by weight in the total composition. Preferably, the amount of metformin hydrochloride is 80.0% to 90.0% by weight in the total composition.

According to an embodiment of the present invention, the amount of empagliflozin is 0.1% to 5.0% by weight in the total composition. Preferably, the amount of empagliflozin is 0.3% to 2.5% by weight in the total composition.

According to one embodiment of the present invention, a solvent comprising alcohol is ethyl alcohol-water mixture or methanol-water mixture or isopropyl alcohol-water mixture.

According to one embodiment of the present invention, an intragranular composition further comprises at least one filler.

Suitable fillers are selected from the group comprising corn starch, microcrystalline cellulose, lactose, anhydrous lactose, starch, mannitol, calcium hydrogen phosphate dihydrate, dicalcium hydrogen phosphate anhydrate, calcium phosphate trihydrate, neutral pellets, magnesium carbonate, magnesium oxide, maltodextrin, maltose, medium chain triglycerides or mixtures thereof.

According to an embodiment of the present invention, the filler is corn starch. The filler helps to provide flowability and compressibility of the powder mixture.

According to an embodiment of the present invention, the amount of filler is 1.0% to 12.0% by weight in the total composition. Preferably, the amount of filler is 2.0% to 8.0% by weight in the total composition.

According to one embodiment of this invention, the tablet further comprises an extragranular composition comprising at least one lubricant and at least one glidant.

Suitable glidants are selected from the group comprising anhydrous colloidal silicon dioxide, sodium stearyl fumarate, magnesium oxide, silicone dioxide, talc, polyethylene glycol, aluminum silicate, magnesium silicate or mixtures thereof.

According to an embodiment of the present invention, the glidant is anhydrous colloidal silicon dioxide. The glidant helps to provide the desired flowability and compressibility of tablet.

According to an embodiment of the present invention, the amount of glidants is 0.1% to 8.0% by weight in the total composition.

Suitable lubricants are selected from the group comprising magnesium stearate, calcium stearate, potassium stearate, stearic acid, sodium chloride, sodium benzoate, sodium acetate, sodium oleate, polyethylene glycols or mixtures thereof.

According to one embodiment of the present invention, the lubricant is magnesium stearate.

According to an embodiment of the present invention, the amount of lubricant is 0.1% to 5.0% by weight in the total composition.

According to an embodiment of the present invention, the film coated tablet is coated with at least one film coating agent.

Suitable film coating agents are selected from the group comprising polymethacrylates, hydroxypropyl methylcellulose, lactose monohydrate, talc, hydroxypropyl cellulose, polyvinyl alcohol (PVA), polyethylene glycol (PEG), glycerin, polyvinyl alcohol-polyethylene glycol copolymers (Kollicoat^{®} IR), ethylcellulose dispersions (Surelease^{®}), polyvinylprolidone, polyvinylprolidone-vinyl acetate copolymer (PVP-VA), iron oxide yellow, iron oxides, all kinds of Opadry^{®}, pigments, dyes, titanium dioxide, coloring agent or mixtures thereof.

According to an embodiment of the present invention, the film coated tablet comprises;
a) 70.0% to 90.0% by weight of Metformin HCl
b) 0.1% to 5.0% by weight of Empagliflozin
c) 2.0% to 15.0% by weight of binder
d) 1.0% to 12.0% by weight of filler
e) 0.1% to 8.0% by weight of glidant
f) 0.1% to 5.0% by weight of lubricant.

According to an embodiment of the present invention, the film coated tablet comprises;
a) Metformin HCl
b) Empagliflozin
c) Corn starch
d) Hydroxypropyl Cellulose
e) Anhydrous colloidal silicon dioxide
f) Magnesium stearate and the film coated is obtained by wet granulation with a solvent comprising alcohol.

According to one embodiment of the present invention, a process for the preparation of the film coated tablet comprises the following steps:
a) Mixing Metformin HCl, empagliflozin, corn starch and hydroxypropyl Cellulose
b) Granulating the mixture with a solvent comprising alcohol,
c) Drying the wet granules,
d) Adding anhydrous colloidal silicon dioxide and magnesium stearate and then mixing,
e) Compressing the mixture into the tablet,
f) Coating the tablets with film coating agent.

In this present invention, a desired flowability and compressibility and a desired content uniformity of the film coated tablet is obtained and it has a simple and low-cost preparation process, in favor of industrial production.

### Example 1: The tablet formulation

| **Ingredients** | **% by weight** |
|---|---|
| Metformin HCl | 80.0 - 90.0 |
| Empagliflozin | 0.3 - 2.5 |
| Corn starch | 2.0 - 8.0 |
| Hydroxypropyl Cellulose | 2.0 - 10.0 |
| Anhydrous colloidal silicon dioxide | 0.1 - 8.0 |
| Magnesium stearate | 0.1 - 5.0 |
| Coating | 0.5 - 3.0 |
| **TOTAL** | **100** |

A process for example 1;
a) Sieving metformin HCl through 2 mm,
b) Mixing Metformin HCl, empagliflozin, corn starch and hydroxypropyl Cellulose or hydroxypropyl methylcellulose,
c) Granulating the mixture with a solvent comprising alcohol,
d) Sieving the wet granule through 8 mm,
e) Drying the wet granules at 50 °C and sieving through 1.5 mm,
f) Adding anhydrous colloidal silicon dioxide and magnesium stearate and then mixing,
g) Compressing the mixture into the tablet,
h) Coating the tablets with film coating agent.

## Claims

1. A film coated tablet comprising an intragranular composition comprising empagliflozin and metformin hydrochloride and at least one binder, wherein the intragranular composition is obtained by wet-granulation using a solvent comprising alcohol.

2. The film coated tablet according to claim 1, wherein binders are selected from the group comprising hydroxypropyl cellulose, hydroxypropyl methylcellulose, pregelatinized starch, polyvinylpyrrolidone, carboxymethylcellulose sodium, cellulose acetate phthalate, chitosan, dextrose, ethylcellulose, glyceryl behenate, hydroxyethyl cellulose, hydroxyethylmethyl cellulose, hydroxypropyl starch, magnesium aluminum silicate, poloxamer, polycarbophil, polydextrose, polyethylene oxide, polymethacrylates, polyoxyethilene-alkyl ethers or mixtures thereof.

3. The film coated tablet according to claim 1, wherein the binder is hydroxypropyl methyl cellulose or hydroxypropyl cellulose.

4. The film coated tablet according to claim 1, wherein the amount of binders is 2.0% to 15.0% by weight in the total composition.

5. The film coated tablet according to claim 1, wherein the solvent comprising alcohol is ethyl alcohol-water mixture or methanol-water mixture or isopropyl alcohol-water mixture.

6. The film coated tablet according to claim 1, wherein an intragranular composition further comprises at least one filler.

7. The film coated tablet according to claim 6, wherein fillers are selected from the group comprising corn starch, microcrystalline cellulose, lactose, anhydrous lactose, starch, mannitol, calcium hydrogen phosphate dihydrate, dicalcium hydrogen phosphate anhydrate, calcium phosphate trihydrate, neutral pellets, magnesium carbonate, magnesium oxide, maltodextrin, maltose, medium chain triglycerides or mixtures thereof.

8. The film coated tablet according to claim 7, wherein the filler is corn starch.

9. The film coated tablet according to claim 8, wherein the amount of filler is 1.0% to 12.0% by weight in the total composition.

10. The film coated tablet according to claim 6, wherein further comprising an extragranular composition comprising at least one lubricant and at least one glidant.

11. The film coated tablet according to claim 10, wherein glidants are selected from the group comprising anhydrous colloidal silicon dioxide, sodium stearyl fumarate, magnesium oxide, silicone dioxide, talc, polyethylene glycol, aluminum silicate, magnesium silicate or mixtures thereof.

12. The film coated tablet according to claim 10, wherein lubricants are selected from the group comprising magnesium stearate, calcium stearate, potassium stearate, stearic acid, sodium chloride, sodium benzoate, sodium acetate, sodium oleate, polyethylene glycols or mixtures thereof.

13. The film coated tablet according to claim 10, wherein the film coated tablet comprises
a) 70.0% to 90.0% by weight of Metformin HCl
b) 0.1% to 5.0% by weight of Empagliflozin
c) 2.0% to 15.0% by weight of binder
d) 1.0% to 12.0% by weight of filler
e) 0.1% to 8.0% by weight of glidant
f) 0.1% to 5.0% by weight of lubricant.

14. The film coated tablet according to claim 10, wherein the film coated tablet comprises:
a) Metformin HCl
b) Empagliflozin
c) Corn starch
d) Hydroxypropyl Cellulose
e) Anhydrous colloidal silicon dioxide
f) Magnesium stearate and the film coated is obtained by wet granulation with a solvent comprising alcohol.

15. A process for the preparation of the film coated tablet according to claims 1-14 comprising the following steps:
a) Mixing Metformin HCl, empagliflozin, corn starch and hydroxypropyl Cellulose
b) Granulating the mixture with a solvent comprising alcohol,
c) Drying the wet granules,
d) Adding anhydrous colloidal silicon dioxide and magnesium stearate and then mixing,
e) Compressing the mixture into the tablet,
f) Coating the tablets with film coating agent.

## Patentansprüche

1. Eine Filmtablette, umfassend eine intragranuläre Zusammensetzung, die Empagliflozin und Metforminhydrochlorid sowie mindestens ein Bindemittel enthält, wobei die intragranuläre Zusammensetzung durch Nassgranulierung unter Verwendung eines Lösungsmittels, das Alkohol enthält, erhalten wird.

2. Filmtablette nach Anspruch 1, wobei die Bindemittel aus der Gruppe ausgewählt sind, die Hydroxypropylcellulose, Hydroxypropylmethylcellulose, vorgelatinierte Stärke, Polyvinylpyrrolidon, Natriumcarboxymethylcellulose, Celluloseacetatphthalat, Chitosan, Dextrose, Ethylcellulose, Glycerylbehenat, Hydroxyethylcellulose, Hydroxyethylmethylcellulose, Hydroxypropylstärke, Magnesiumaluminiumsilikat, Poloxamer, Polycarbophil, Polydextrose, Polyethylenoxid, Polymethacrylate, Polyoxyethylen-Alkylether oder Mischungen davon ausgewählt sind.

3. Filmtablette nach Anspruch 1, wobei das Bindemittel Hydroxypropylmethylcellulose oder Hydroxypropylcellulose ist.

4. Filmtablette nach Anspruch 1, wobei der Anteil der Bindemittel 2,0 bis 15,0 Gew.-% der Gesamtzusammensetzung beträgt.

5. Filmtablette nach Anspruch 1, wobei das Alkohol enthaltende Lösungsmittel ein Ethylalkohol-Wasser-Gemisch oder ein Methanol-Wasser-Gemisch oder ein Isopropylalkohol-Wasser-Gemisch ist.

6. Filmtablette nach Anspruch 1, wobei eine intragranulare Zusammensetzung ferner mindestens einen Füllstoff umfasst.

7. Filmtablette nach Anspruch 6, wobei die Füllstoffe aus der Gruppe ausgewählt sind, die Maisstärke, mikrokristalline Cellulose, Laktose, wasserfreie Laktose, Stärke, Mannitol, Calciumhydrogenphosphat-Dihydrat, Dicalciumhydrogenphosphat-Anhydrat, Calciumphosphat-Trihydrat, neutrale Pellets, Magnesiumcarbonat, Magnesiumoxid, Maltodextrin, Maltose, mittelkettige Triglyceride oder Mischungen davon umfasst.

8. Filmtablette nach Anspruch 7, wobei der Füllstoff Maisstärke ist.

9. Filmtablette nach Anspruch 8, wobei die Menge an Füllstoff 1,0 bis 12,0 Gew.-% der Gesamtzusammensetzung beträgt.

10. Filmtablette nach Anspruch 6, wobei sie ferner eine extragranulare Zusammensetzung umfasst, die mindestens ein Schmiermittel und mindestens ein Fließregulierungsmittel enthält.

11. Filmtablette nach Anspruch 10, wobei die Fließregulierungsmittel aus der Gruppe ausgewählt sind, die aus wasserfreiem kolloidalem Siliciumdioxid, Natriumstearylfumarat, Magnesiumoxid, Siliciumdioxid, Talk, Polyethylenglykol, Aluminiumsilikat, Magnesiumsilikat oder Mischungen davon besteht.

12. Filmtablette nach Anspruch 10, wobei die Schmiermittel aus der Gruppe ausgewählt sind, die Magnesiumstearat, Calciumstearat, Kaliumstearat, Stearinsäure, Natriumchlorid, Natriumbenzoat, Natriumacetat, Natriumoleat, Polyethylenglykole oder Mischungen davon umfasst.

13. Filmtablette nach Anspruch 10, wobei die Filmtablette umfasst
a) 70,0 bis 90,0 Gew.-% Metformin-HCl
b) 0,1 bis 5,0 Gew.-% Empagliflozin
c) 2,0 bis 15,0 Gew.-% Bindemittel
d) 1,0 bis 12,0 Gew.-% Füllstoff
e) 0,1 bis 8,0 Gew.-% Fließregulierungsmittel
f) 0,1 bis 5,0 Gew.-% Schmiermittel.

14. Filmtablette nach Anspruch 10, wobei die Filmtablette umfasst:
a) Metformin-HCl
b) Empagliflozin
c) Maisstärke
d) Hydroxypropylcellulose
e) wasserfreies kolloidales Siliciumdioxid
f) Magnesiumstearat, wobei die Filmtablette durch Nassgranulierung mit einem alkoholhaltigen Lösungsmittel hergestellt wird.

15. Verfahren zur Herstellung der Filmtablette gemäß den Ansprüchen 1-14, umfassend die folgenden Schritte:
a) Mischen von Metformin-HCl, Empagliflozin, Maisstärke und Hydroxypropylcellulose
b) Granulieren der Mischung mit einem alkoholhaltigen Lösungsmittel,
c) Trocknen des Nassgranulats,
d) Zugabe von wasserfreiem kolloidalem Siliciumdioxid und Magnesiumstearat und anschließendes Mischen,
e) Verpressen der Mischung zu einer Tablette,
f) Beschichtung der Tabletten mit einem Filmüberzugsmittel.

## Revendications

1. Comprimé pelliculé comprenant une composition intragranulaire comprenant de l'empagliflozine, du chlorhydrate de metformine et au moins un liant, dans lequel la composition intragranulaire est obtenue par granulation humide en utilisant un solvant comprenant de l'alcool.

2. Comprimé pelliculé selon la revendication 1, dans lequel les liants sont choisis dans le groupe comprenant l'hydroxypropylcellulose, l'hydroxypropylméthylcellulose, l'amidon prégélatinisé, la polyvinylpyrrolidone, la carboxyméthylcellulose sodique, l'acétophtalate de cellulose, le chitosan, le dextrose, l'éthylcellulose, le béhénate de glycéryle, l'hydroxyéthylcellulose, l'hydroxyéthylméthylcellulose, l'hydroxypropylamidon, le silicate de magnésium et d'aluminium, le poloxamère, le polycarbophile, le polydextrose, l'oxyde de polyéthylène, les polyméthacrylates, les éthers alkyliques de polyoxyéthylène ou leurs mélanges.

3. Comprimé pelliculé selon la revendication 1, dans lequel le liant est l'hydroxypropylméthylcellulose ou l'hydroxypropylcellulose.

4. Comprimé pelliculé selon la revendication 1, dans lequel la quantité de liants est de 2,0 % à 15,0 % en poids dans la composition totale.

5. Comprimé pelliculé selon la revendication 1, dans lequel le solvant comprenant de l'alcool est un mélange d'alcool éthylique et d'eau, ou un mélange de méthanol et d'eau, ou un mélange d'alcool isopropylique et d'eau.

6. Comprimé pelliculé selon la revendication 1, dans lequel la composition intragranulaire comprend en outre au moins un agent de charge.

7. Comprimé pelliculé selon la revendication 6, dans lequel les agents de charge sont choisis dans le groupe comprenant l'amidon de maïs, la cellulose microcristalline, le lactose, le lactose anhydre, l'amidon, le mannitol, l'hydrogénophosphate de calcium dihydraté, l'hydrogénophosphate de calcium anhydre, le phosphate de calcium trihydraté, les pellets neutres, le carbonate de magnésium, l'oxyde de magnésium, la maltodextrine, le maltose, les triglycérides à chaîne moyenne ou leurs mélanges.

8. Comprimé pelliculé selon la revendication 7, dans lequel l'agent de charge est l'amidon de maïs.

9. Comprimé pelliculé selon la revendication 8, dans lequel la quantité d'agent de charge est de 1,0 % à 12,0 % en poids dans la composition totale.

10. Comprimé pelliculé selon la revendication 6, dans lequel celui-ci comprend en outre une composition extragranulaire comprenant au moins un lubrifiant et au moins un glissant.

11. Comprimé pelliculé selon la revendication 10, dans lequel les glissants sont choisis dans le groupe comprenant le dioxyde de silicium colloïdal anhydre, le fumarate de stéaryle sodique, l'oxyde de magnésium, le dioxyde de silicium, le talc, le polyéthylène glycol, le silicate d'aluminium, le silicate de magnésium ou leurs mélanges.

12. Comprimé pelliculé selon la revendication 10, dans lequel les lubrifiants sont choisis dans le groupe comprenant le stéarate de magnésium, le stéarate de calcium, le stéarate de potassium, l'acide stéarique, le chlorure de sodium, le benzoate de sodium, l'acétate de sodium, l'oléate de sodium, les polyéthylène glycols ou leurs mélanges.

13. Comprimé pelliculé selon la revendication 10, dans lequel le comprimé pelliculé comprenant
a) 70,0 % à 90,0 % en poids de metformine HCl
b) 0,1 % à 5,0 % en poids d'empagliflozine
c) 2,0 % à 15,0 % en poids de liant
d) 1,0 % à 12,0 % en poids d'agent de charge
e) 0,1 % à 8,0 % en poids de glissant
f) 0,1 % à 5,0 % en poids de lubrifiant.

14. Comprimé pelliculé selon la revendication 10, dans lequel le comprimé pelliculé comprenant :
a) de la metformine HCl
b) de l'empagliflozine
c) de l'amidon de maïs
d) de l'hydroxypropylcellulose
e) du dioxyde de silicium colloïdal anhydre
f) du stéarate de magnésium et le comprimé pelliculé est obtenu par granulation humide avec un solvant comprenant de l'alcool.

15. Procédé de préparation du comprimé pelliculé selon les revendications 1-14, comprenant les étapes suivantes :
a) le mélange de metformine HCl, d'empagliflozine, d'amidon de maïs et d'hydroxypropylcellulose,
b) la granulation du mélange avec un solvant comprenant de l'alcool,
c) le séchage des granules humides,
d) l'ajout de dioxyde de silicium colloïdal anhydre et de stéarate de magnésium, puis le mélange,
e) la compression du mélange sous forme de comprimé,
f) l'enrobage des comprimés avec un agent de pelliculage.
